# FASCICULE DE BREVET EUROPEEN

(11) **EP 0 751 110 B1**
(45) Date de publication et mention de la délivrance du brevet: **11.09.2002**
(21) Numéro de dépôt: 96401436.9
(22) Date de dépôt: 28.06.1996
(51) Int. Cl.: C07C 45/78, C07C 45/81, C07C 47/22

(54) **Procédé de purification d'un flux gazeux contenant de l'acroléine**
Verfahren zur Reinigung von einem Acrolein enthaltenden Gasstromes
Process for the purification of a gaseous stream containing acrolein

(30) Priorité: 29.06.1995 FR 9507858
(43) Date de publication de la demande: 02.01.1997
(73) Titulaire: AVENTIS ANIMAL NUTRITION S.A., 92160 Antony (FR)
(72) Inventeur: Hego, Michel, 38800 Le Pont de Claix (FR); Kress, Frédéric, 38200 Vienne (FR)
(74) Mandataire: Mérigeault, Shona

(56) Documents cités:
- EP-A- 0 559 227
- FR-A- 2 287 437
- US-A- 5 352 837

## Description

La présente invention concerne un procédé de purification d'un flux gazeux comprenant de l'acroléine, ainsi que de l'eau, des acides et des gaz inertes, pour en extraire l'acroléine.

L'acroléine est une matière première, dont une utilisation majeure à l'échelle industrielle est la synthèse de l'aldéhyde bêta-méthylthiopropionique (AMTP) par réaction d'acroléine avec du méthylmercaptan.

Elle est produite par oxydation du propylène en phase gazeuse par l'air en présence d'eau. Classiquement, le mélange de production d'une installation d'oxydation du propylène en phase gazeuse est un mélange gazeux contenant de l'acroléine en une proportion supérieure à 10% en poids; des gaz inertes tels que de l'azote, de l'oxygène, du monoxyde et du dioxyde de carbone ; du propylène ; de l'eau; et des sous produits de réaction tels que des acides acrylique, acétique et formique, du formaldéhyde, de l'acétaldéhyde, de l'alcool allylique, ainsi que des polymères résultant de la dégradation de l'acroléine et de l'acide acrylique en particulier.

En vue de la mise en oeuvre de l'acroléine dans une réaction telle que la synthèse directe de l'AMTP, il faut séparer de ce mélange complexe au moins en partie l'eau, les impuretés acides, notamment l'acide acrylique, de même que les produits lourds que sont les polymères. On entend par synthèse directe de l'AMTP un des procédés tels que décrits notamment dans les brevets US 4 225 516 ou US 5 352 837. Ce procédé consiste à faire réagir le gaz contenant l'acroléine ainsi que la source de méthylmercaptan dans une solution d'aldéhyde méthylthiopropionique. En particulier, les spécifications en eau et en acides tels que notamment l'acide acrylique, dans le mélange gazeux purifié utilisable pour la réaction ultérieure sont très importantes car elles conditionnent les performances de ladite réaction.

Plusieurs méthodes dans le but de purifier l'acroléine sont connues et exploitées.

La première méthode selon le brevet US 4 219 389 consiste à extraire les acides contenus dans le mélange gazeux par une absorption dans un solvant organique (notamment l'éthyl-2-hexanol, ou le tributyl phosphate). Cependant, ce type de méthode présente l'inconvénient de nécessiter l'emploi d'un solvant qu'il est nécessaire de purifier en vue de le recycler dans le processus, augmentant de ce fait les coûts du procédé.

La deuxième méthode de purification de l'acroléine, par exemple selon le brevet US 3 433 840 consiste à extraire les acides contenus dans le mélange gazeux par une absorption sélective à l'eau, puis à récupérer l'acroléine contenue dans le flux gazeux chargé d'eau et d'incondensables provenant de l'étape d'absorption. Une seconde étape consiste à distiller le mélange liquide résultant pour obtenir l'azéotrope acroléine/eau. Cependant, ce type de procédé a l'inconvénient de nécessiter des quantités importantes d'eau pour l'absorption. Il présente également des risques importants de dégradation de l'acroléine et de polymérisation lors de l'étape de distillation.

Une autre voie a été développée en vue de la synthèse directe d'AMTP. Le mélange issu de l'oxydation du propylène est tout d'abord épuré pour éliminer le maximum de composés acides, comme précédemment par absorption par l'eau. Après condensation de l'eau, l'acroléine est extraite du gaz résiduel par absorption dans de l'AMTP froid provenant d'un réacteur de production d'AMTP. La solution d'acroléine dans l'AMTP ainsi obtenue est ensuite engagée directement dans le réacteur de production d'AMTP avec du méthylmercaptan. Un tel procédé est décrit dans le brevet US 4 225 516 mentionné précédemment. L'épuration préalable, en ce qui concerne les acides, du mélange de départ contenant l'acroléine reste toutefois nécessaire et on retrouve donc les inconvénients mentionnés auparavant.

Un autre procédé de purification connu de l'acroléine décrit dans la demande de brevet EP-A-0 559 227 consiste à refroidir le mélange réactionnel dans une tour de refroidissement où il est mis en contact avec du liquide de condensation, un gaz effluent contenant majoritairement des incondensables et de l'acroléine étant récupéré en tête de la tour. Dans les conditions indiquées, où le condensat en pied de tour a une température de 35 à 50°C, le gaz effluent en tête de tour a une température de 35 à 55°C et où le temps de séjour du condensat dans la tour est réduit, l'épuration du mélange réactionnel provoque l'absorption d'une quantité relativement importante d'acroléine dans le condensat, selon une teneur d'environ 3 % en poids.

Le brevet français FR 2 287 437 décrit lui aussi un procédé de purification de l'acide acrylique dans lequel le gaz contenant le mélange acroléine/acide acrylique est mis en contact avec un courant descendant de liquide que l'on retire partiellement à titre de condensat d'acide acrylique et on recycle le reste à titre de liquide de recyclage. Les vapeurs de tête contiennent de l'acroléine et de l'acide acrylique. Le courant liquide étant à une température ne s'écartant pas de plus de 15°C du point de rosée de l'effluent gazeux. Les vapeurs de tête contenant de l'acroléine et de l'acide acrylique étant retirées à une température comprise entre 0 et 40°C.

Dans la présente invention l'objet consiste à purifier l'acroléine et non l'acide acrylique.

Là présente invention a donc pour but d'obvier aux inconvénients des procédés connus et de proposer un procédé de purification d'un mélange gazeux comprenant de l'acroléine, de l'eau, des acides et des incondensables, afin de permettre l'utilisation directe de l'acroléine notamment par la synthèse de l'AMTP.

La présente invention a de même pour objectif de produire de l'acroléine dans des conditions telles que le rendement de purification soit le plus élevé possible et en minimisant les risques de dégradation de l'acroléine.

Enfin, la présente invention a donc pour but d'éviter l'encrassement des appareillages mis en oeuvre dans le procédé.

A cet effet, l'invention a pour objet un procédé de purification d'un flux gazeux d'alimentation comprenant de l'acroléine ainsi que de l'eau, des acides et des gaz inertes, provenant notamment de l'oxydation du propylène en acroléine en phase gazeuse, selon lequel ledit flux gazeux d'alimentation est, dans une première étape, fractionné en un effluent gazeux et un flux liquide dans une colonne de refroidissement, fonctionnant de telle sorte que la température du flux liquide en pied de colonne soit inférieure ou égale au point de rosée dudit flux gazeux d'alimentation, l'écart de températures ne dépassant pas 20°C, de préférence ne dépassant pas 10°C, et, dans une deuxième étape, ledit effluent gazeux est ensuite condensé à une température inférieure à 20°C pour donner une fraction liquide et une fraction gazeuse purifiée.

Les acides contenus dans le flux gazeux d'alimentation sont des acides organiques. On citera en particulier l'acide acrylique, l'acide formique, l'acide acétique ou l'acide maléique.

Par gaz inertes, on comprend tous les composés gazeux qui restent dans la phase gazeuse du début à la fin du procédé de production de l'invention et que l'on retrouve dans la fraction gazeuse purifiée, après l'étape de condensation. A cet égard, les gaz inertes du mélange à purifier pourront par la suite être occasionnellement dénommés "incondensables" puisqu'ils ne sont pas condensés dans les conditions de température et de pression mises en oeuvre dans le procédé de l'invention. Il peut s'agir notamment d'azote, d'oxygène et d'autres gaz de l'air, d'oxydes de carbone, ou de propylène.

Grâce au procédé de l'invention, on peut en fin de la deuxième étape obtenir une fraction gazeuse purifiée contenant de l'acroléine et des incondensables, dont la teneur pondérale en eau est inférieure ou égale à 2 % et la teneur pondérale en acides est inférieure ou égale à 100 ppm.

Dans la première étape selon le procédé de l'invention, le flux gazeux d'alimentation qui provient de l'oxydation du propylène en phase gazeuse, est de préférence refroidi à partir de sa température de production jusqu'à une température de 100 à 200°C, et introduit dans la partie basse de la colonne de refroidissement.

Le flux d'alimentation de la colonne de refroidissement contient notamment entre 10 et 15 % d'acroléine et environ 20 à 30 % d'eau et moins de 5 % d'acide acrylique, de préférence moins de 2 %, le complément étant constitué des gaz incondensables et de divers constituants organiques issus de l'oxydation du propylène.

La circulation du flux gazeux dans la colonne à contre-courant avec un liquide froid provoque la condensation de l'eau et de l'acide acrylique ainsi que des autres composants condensables éventuellement présents. Le liquide condensé redescend par gravité jusqu'au pied de la colonne. Les gaz de tête de la colonne sont appauvris en impuretés. Ils sont constitués essentiellement d'acroléine et de gaz incondensables.

De préférence, la température des gaz de tête est de 30 à 60°C, et encore plus préférentiellement entre 50 et 60°C.

La température du flux liquide en pied de colonne est inférieure de moins de 20°C, de préférence de moins de 10°C, au point de rosée du flux gazeux d'alimentation. Très avantageusement, la température du flux liquide en pied de colonne est sensiblement égale au point de rosée du mélange gazeux introduit dans la colonne pour minimiser la condensation de l'acroléine ainsi que sa dégradation. Le plus souvent, elle est inférieure à 100°C. Le point de rosée du mélange provenant de l'oxydation catalytique du propylène, et contenant environ 10 à 15 % d'acroléine et 20 à 30 % d'eau, est compris entre 70 et 90°C, pour une pression d'environ 1,2.10⁵ Pa.

Avantageusement, la colonne de refroidissement fonctionne sous une pression de 10⁵ à 3.10⁵ Pa.

De préférence, le temps de séjour de l'acroléine dans la colonne de refroidissement est de 5 à 10 minutes, ce par quoi on limite les risques de dégradation tout en séparant efficacement les impuretés grâce au procédé de l'invention.

Dans le procédé de l'invention, de manière avantageuse, une partie dudit flux liquide accumulé dans le pied de la colonne de refroidissement est prélevée, refroidie, et est recyclée en tant que liquide froid circulant dans la colonne de refroidissement. Il est possible d'ajouter au flux liquide recyclé un inhibiteur de polymérisation de l'acroléine et de l'acide acrylique. Celui-ci peut notamment être choisi parmi l'hydroquinone, la phénothiazine et ses dérivés.

Le flux recyclé contient notamment les acides organiques dont l'acide acrylique et moins de 2 % en poids d'acroléine, de préférence moins de 1,5 %, et au moins 90 % d'eau.

Le flux liquide recyclé peut être introduit en un ou plusieurs endroits de la colonne de refroidissement. De préférence, il est injecté en tête de colonne pour arroser le mélange gazeux à traiter. Plus particulièrement, le liquide recyclé est refroidi entre 15 et 45°C par un réfrigérant à eau industrielle ou un réfrigérant à eau glycolée.

Selon une étape facultative du procédé de l'invention, une autre partie du flux liquide accumulé dans la colonne de refroidissement peut être prélevée pour subir une opération de strippage, de manière à recycler au pied de ladite colonne une partie de l'acroléine entraînée sous forme gazeuse.

Avantageusement, le flux liquide prélevé à cet effet est réchauffé à une température de 90 à 120°C avant le strippage.

L'opération facultative de strippage est réalisée en faisant circuler dans une colonne le flux liquide prélevé, à contre-courant avec un gaz inerte, notamment de l'azote, le liquide étant introduit en tête de la colonne et le gaz étant introduit dans la partie basse de la colonne. Avantageusement, la température de ce gaz de strippage est de 130 à 160°C, de préférence environ 150°C, afin d'éliminer tout risque de dégradation de l'acroléine et d 'améliorer l'efficacité du strippage.

Au cours de l'opération, l'acroléine contenue dans le flux liquide est extraite par le gaz de strippage, de sorte que l'on récupère un flux gazeux contenant de l'acroléine, une faible partie de l'eau et les incondensables, alors qu'il reste les acides et la majorité de l'eau en phase liquide.

De préférence, la pression dans la colonne de strippage est supérieure à la pression dans la colonne de refroidissement, pour permettre la réinjection du flux gazeux issu du strippage dans la colonne de refroidissement avec le flux gazeux d'alimentation. Il est à noter que la pression de la colonne de strippage ne doit pas être trop élevée pour ne pas être préjudiciable à l'efficacité du strippage.

Le retour de ce flux gazeux issu du strippage peut être réalisé par mélange dudit flux de strippage avec le flux gazeux d'alimentation ou, de préférence, par injection dudit flux de strippage dans la colonne de refroidissement, environ au même niveau que l'introduction du flux gazeux d'alimentation.

Dans la deuxième étape du procédé selon l'invention, on condense en partie le gaz issu de la tête de la colonne de refroidissement, de préférence dans un condenseur à surface.

La séparation de la phase liquide condensée fournit un gaz appauvri en impuretés, notamment en eau et en acides, et contenant de l'acroléine et les incondensables.

Avantageusement, ledit effluent gazeux est condensé une ou plusieurs fois de suite pour fournir une fraction gazeuse contenant l'acroléine, la température du gaz en sortie du dernier condenseur étant inférieure à 20°C.

De préférence, la condensation de l'effluent gazeux se fait en présence d'un inhibiteur de polymérisation de l'acroléine.

A l'issue de la ou des opérations de condensation, la fraction gazeuse contenant l'acroléine peut être récupérée en tant que gaz purifié qui peut être mis en oeuvre directement pour la synthèse de l'AMTP.

Avantageusement, selon une étape supplémentaire facultative, ladite fraction gazeuse peut être soumise à une opération d'absorption par circulation à contre-courant avec de l'eau, pour éliminer une quantité supplémentaire d'acides résiduaires. Cette opération peut être mise en oeuvre dans une colonne d'absorption. L'eau circule à contre-courant de ladite fraction gazeuse à une température inférieure à la température de ladite fraction, avec un débit massique tel que le rapport du débit massique d'eau au débit massique de la fraction gazeuse soit compris entre 0,005 et 0,05, de préférence entre 0,01 et 0,05. On obtient ainsi avantageusement un gaz purifié dont la teneur en eau est inférieure à 2 % en poids, et de préférence inférieure ou égale à 1 % en poids, et la teneur en acides est inférieure à 100 ppm.

Les gammes mentionnées pour le rapport du débit d'eau au débit de la fraction gazeuse ont été exprimées pour une absorption effectuée dans une colonne comprenant 5 à 10 plateaux théoriques.

La mise en oeuvre d'une colonne présentant un nombre de plateaux en dehors des valeurs mentionnées est possible. Dans ce cas, l'homme du métier est à même, avec ses connaissances, d'adapter les débits d'eau et de fraction gazeuse en conséquence.

Au cours de l'opération d'absorption par l'eau, la dissolution d'espèces chimiques dans l'eau a toutefois pour effet de réchauffer le gaz sortant de la colonne d'absorption.

Aussi, pour augmenter l'efficacité de l'absorption à l'eau, et donc respecter les teneurs en eau et en acides désirées dans le gaz purifié, il est préférable que le gaz soit le plus froid possible. Par conséquent on préfère sous-refroidir le gaz dans l'opération de condensation.

De même que précédemment, on peut additionner un inhibiteur de polymérisation de l'acroléine dans l'eau d'absorption.

Une teneur en eau dans le gaz purifié contenant l'acroléine inférieure à 2% en poids, et une teneur totale en acides inférieure à 100 ppm, sont des valeurs préférées pour l'utilisation dudit gaz comme matériau de départ pour la synthèse directe de l'AMTP.

Les exemples 1 et 2 suivants illustrent deux applications pratiques du procédé de l'invention.

### EXEMPLE 1

Une installation du type représenté sur la figure 1, comprenant un condenseur en tant qu'appareil de condensation, est utilisé pour purifier un mélange gazeux provenant d'un réacteur d'oxydation du propylène en acroléine en phase vapeur. La composition du mélange est la suivante.

(Les proportions sont indiquées par rapport au poids du mélange).

| | |
|---|---|
| Gaz incondensables (azote, oxygène, propylène, oxydes de carbone) | 63,3 % |
| Eau | 21,0 % |
| Acroléine | 12,3 % |
| Acide acrylique | 1,4 % |
| Autres (acétaldéhyde, alcool allylique, formaldéhyde...) | 2,0 % |

Le mélange gazeux à purifier est introduit à la température de 180°C au pied d'une colonne de refroidissement à 10 plateaux à trous, sous le dernier plateau, avec un débit massique de 20 kg/h, à la pression de 1,2.10⁵ Pa.

Le liquide de pied de colonne est maintenu à une température de 70,3 °C, alors que le point de rosée du flux gazeux d'alimentation est de 74,7°C (différence égale à 4,3°C). Il contient 1,3 % en poids d'acroléine.

Le recyclage de liquide de pied en tête de colonne se fait à un débit de 80 kg/h, l'échangeur de chaleur fournissant un liquide recyclé à une température de 30°C.

Une autre partie du liquide de pied de colonne est envoyée dans la colonne de strippage à l'azote, remplie de garnissage vrac, fonctionnant sous une température de 90°C avec un débit massique d'azote de strippage de 0,6 kg/h. On en récupère un liquide ne contenant plus que 100 ppm d'acroléine.

A la sortie de la colonne de refroidissement, les gaz de tête sont refroidis à 2°C dans le condenseur. La fraction gazeuse récupérée en sortie du condenseur est analysée pour déterminer sa teneur en acides et en eau.

Le dosage des acides organiques est effectué par chromatographie ionique après barbotage du gaz dans l'eau.

Le dosage de l'eau est réalisé par la méthode de Karl Fischer après barbotage du gaz dans le n-propanol.

On détermine que le gaz purifié contient 96 ppm d'acides et 0,34 % d'eau par rapport au poids total dudit gaz.

### EXEMPLE 2

Une installation du type représenté sur la figure 2, comprenant les différents éléments de l'installation de l'exemple 1 et en outre une colonne d'absorption à six plateaux à trous, est utilisée pour purifier le même mélange que celui de l'exemple 1.

Les paramètres de fonctionnement de l'exemple 1 sont conservés hormis que le condenseur refroidit à 4°C les gaz de tête de la colonne de refroidissement.

La fraction gazeuse récupérée en sortie du condenseur est introduite en pied de la colonne d'absorption avec un débit de 16,2 kg/h. Dans cette colonne d'absorption circule un courant d'eau introduite à 4°C au-dessus du premier plateau de la colonne, avec un débit de 0,2kg/h.

On détermine comme dans l'exemple 1 que le flux gazeux qui sort de la colonne d'absorption contient 0,43 % d'eau et moins de 10 ppm d'acides par rapport au poids total dudit flux gazeux.

## Revendications

1. Procédé de purification d'un flux gazeux d'alimentation comprenant de l'acroléine ainsi que de l'eau, des acides et des gaz inertes, provenant notamment de l'oxydation du propylène en acroléine en phase gazeuse, selon lequel, dans une première étape, ledit flux gazeux d'alimentation est fractionné en un effluent gazeux et un flux liquide dans une colonne de refroidissement, fonctionnant de telle sorte que la température du flux liquide en pied de colonne soit inférieure ou égale au point de rosée dudit flux gazeux d'alimentation, l'écart de température ne dépassant pas 20°C, et, dans une deuxième étape, l'effluent gazeux précité est ensuite condensé à une température inférieure à 20°C pour donner une fraction liquide et une fraction gazeuse purifiée.

2. Procédé selon la revendication précédente, dans lequel la température du flux liquide en pied de la colonne de refroidissement est inférieure ou égale au point de rosée du flux gazeux d'alimentation, l'écart ne dépassant pas 10°C.

3. Procédé selon l'une quelconque des revendications précédentes, dans lequel le flux gazeux d'alimentation est introduit à une température de 100 à 200°C dans la colonne de refroidissement, la température de gaz en tête de colonne est de 30 à 60°C et la température du flux liquide en pied de colone est voisin du point de rosée du flux gazeux d'alimentation.

4. Procédé selon l'une quelconque des revendications précédentes dans lequel une partie dudit flux liquide accumulé au pied de la colonne de refroidissement est prélevée, éventuellement refroidie, et est recyclé en tant que flux liquide circulant dans la colonne de refroidissement.

5. Procédé selon la revendication 4, dans lequel la température du flux liquide recyclé avant circulation dans la colonne de refroidissement, est de 15 à 45°C.

6. Procédé selon l'une quelconque des revendications précédentes dans lequel la fraction gazeuse purifiée et isolée après la condensation est soumise à une opération d'absorption par circulation à contre-courant de ladite fraction gazeuse avec de l'eau dont la température est inférieure à la température de ladite fraction et avec un débit massique d'eau tel que le rapport du débit massique d'eau au débit massique de la fraction gazeuse soit compris entre 0,005 et 0,5.

7. Procédé selon l'une quelconque des revendications précédentes dans lequel une partie du flux liquide obtenu dans la colonne de refroidissement est soumise à une opération de strippage avec un gaz inerte pour fournir un flux gazeux de strippage contenant de l'acroléine, lequel est introduit dans la colonne de refroidissement précitée avec le flux gazeux d'alimentation.

8. Procédé selon la revendication 7, dans lequel la partie du flux accumulé au pied de la colonne de refroidissement destinée au stripage est prélevée, réchauffée à une température de 90 à 120°C et introduite dans une colonne où elle circule à contre-courant avec un gaz inerte.

9. Procédé selon la revendication 8, dans lequel 1a température du gaz inerte de strippage est de 130 à 160°C.

## Patentansprüche

1. Verfahren zur Reinigung eines zuführenden Gasstroms, der Acrolein sowie Wasser, Säuren und Inertgase enthält, die insbesondere aus der Gasphasenoxidation von Propylen zu Acrolein stammen, wobei nach diesem Verfahren in einer ersten Stufe der zuführende Gasstrom in einer Kühlkolonne in einen Gasstrom und einen Flüssigkeitsstrom fraktioniert wird, wobei die Kühlkolonne so arbeitet, daß die Temperatur des Flüssigkeitsstroms am Boden der Kolonne höchstens dem Taupunkt des zuführenden Gasstroms entspricht, wobei die Temperaturdifferenz 20 °C nicht übersteigt, und in einer zweiten Stufe der obengenannte Gasstrom dann bei einer Temperatur unter 20 °C kondensiert wird, wodurch eine flüssige Fraktion und eine gereinigte gasförmige Fraktion gebildet wird.

2. Verfahren nach dem vorhergehenden Anspruch, wobei die Temperatur des Flüssigkeitsstroms am Boden der Kühlkolonne höchstens dem Taupunkt des zuführenden Gasstroms entspricht, wobei der Unterschied 10 °C nicht übersteigt.

3. Verfahren nach einem der vorhergehenden Ansprüche, wobei der zuführende Gasstrom bei einer Temperatur von 100 bis 200 °C in die Kühlkolonne geleitet wird, die Temperatur des Gases am Kopf der Kolonne 30 bis 60 °C beträgt und die Temperatur des Flüssigkeitsstroms am Boden der Kolonne in der Nähe des Taupunktes des zuführenden Gasstroms liegt.

4. Verfahren nach einem der vorhergehenden Ansprüche, wobei ein Teil des Flüssigkeitsstromes, der sich am Boden der Kühlkolonne angesammelt hat, entnommen, gegebenenfalls abgekühlt und als Flüssigkeitsstrom, der in der Kühlkolonne zirkuliert, rückgeführt wird.

5. Verfahren nach Anspruch 4, wobei die Temperatur des rückgeführten Flüssigkeitsstroms vor der Zirkulation in der Kühlkolonne 15 bis 45 °C ist.

6. Verfahren nach einem der vorhergehenden Ansprüche, wobei die gereinigte gasförmige Fraktion, die nach der Kondensation abgetrennt wurde, durch Zirkulation der gasförmigen Fraktion im Gegenstrom mit Wasser einer Absorptionsbehandlung unterzogen wird, wobei die Temperatur des Wassers unter der Temperatur der Fraktion liegt und wobei der Massestrom des Wassers so gewählt ist, daß das Verhältnis des Massedurchsatzes des Wassers und des Massedurchsatzes der gasförmigen Fraktion im Bereich von 0,005 bis 0,5 liegt.

7. Verfahren nach einem der vorhergehenden Ansprüche, wobei ein Teil des in der Kühlkolonne anfallenden Flüssigkeitsstroms mit einem Inertgas gestrippt wird, wodurch ein Acrolein-haltiger Strippgasfluß gebildet wird, der mit dem zuführenden Gasstrom in die obengenannte Kühlkolonne geleitet wird.

8. Verfahren nach Anspruch 7, wobei der Teil des am Boden der Kühlkolonne anfallenden Stroms, der zum Strippen vorgesehen ist, entnommen wird, auf eine Temperatur von 90 bis 120 °C erwärmt wird und in eine Kolonne geleitet wird, wo er im Gegenstrom mit einem Inertgas zirkuliert.

9. Verfahren nach Anspruch 8, wobei die Temperatur des zum Strippen verwendeten Inertgases im Bereich von 130 bis 160 °C liegt.

## Claims

1. Process for purification of a feed gas stream including acrolein and water, acids and inert gases, originating particularly from the gas-phase oxidation of propylene to acrolein, according to which, in a first stage, the said feed gas stream is fractionated into a gaseous effluent and a liquid stream in a cooling column operating so that the temperature of the liquid stream at the bottom of the column is lower than or equal to the dew point of the said feed gas stream, the difference in temperature not exceeding 20°C and, in a second stage, the abovementioned gaseous effluent is next condensed at a temperature that is lower than 20°C to give a liquid fraction and a purified gaseous fraction.

2. Process according to the preceding claim, in which the temperature of the liquid stream at the bottom of the cooling column is lower than or equal to the dew point of the feed gas stream, the difference not exceeding 10°C.

3. Process according to either of the preceding claims, in which the feed gas stream is introduced at a temperature of 100 to 200°C into the cooling column, the gas temperature at the top of the column is from 30 to 60°C and the temperature of the liquid stream is close to the dew point of the feed gas stream.

4. Process according to any one of the preceding claims, in which a portion of the said liquid stream accumulated at the bottom of the cooling column is abstracted, optionally cooled and is recycled as liquid stream circulating in the cooling column.

5. Process according to claim 4, in which the temperature of the recycled liquid stream, before circulation in the cooling column, is from 15 to 45°C.

6. Process according to any one of the preceding claims, in which the gaseous fraction which is purified and isolated after the condensation is subjected to an operation of absorption by countercurrentwise circulation of the said gaseous fraction with water whose temperature is lower than the temperature of the said fraction and at a mass flow rate of water such that the ratio of the mass flow rate of water to the mass flow rate of the gaseous fraction is between 0.005 and 0.5.

7. Process according to any one of the preceding claims, in which a portion of the liquid stream obtained in the cooling column is subjected to an operation of stripping with an inert gas to provide a stripped gaseous stream containing acrolein, which is introduced into the abovementioned cooling column with the feed gas stream.

8. Process according to claim 7, in which the portion of the liquid stream accumulated at the bottom of the cooling column, intended for stripping, is removed, heated to a temperature of 90 to 120°C and introduced into a column where it travels countercurrentwise with an inert gas.

9. Process according to claim 8, in which the temperature of the inert stripping gas is from 130 to 160°C.
